# EUROPEAN PATENT SPECIFICATION

(11) **EP 4 424 779 B1**
(45) Date of publication and mention of the grant of the patent: **25.11.2026**
(21) Application number: 22887186.9
(22) Date of filing: 28.10.2022
(51) Int. Cl.: C08L 101/14, A61F 13/15, A61F 13/53, A61L 15/18, A61L 15/22, A61L 15/24, A61L 15/42, A61L 15/46, C08K 3/04, A61F 13/84

(54) **WATER-ABSORBING RESIN COMPOSITION, ABSORBER, ABSORBENT ARTICLE, AND METHOD FOR SEPARATION PROCESSING OF WATER-ABSORBING RESIN PARTICLES FROM ABSORBENT ARTICLE**
WASSERABSORBIERENDE HARZZUSAMMENSETZUNG, ABSORBER, SAUGFÄHIGER ARTIKEL UND VERFAHREN ZUR TRENNUNG VON WASSERABSORBIERENDEN HARZPARTIKELN VON SAUGFÄHIGEM ARTIKEL
COMPOSITION DE RÉSINE ABSORBANTE, CORPS ABSORBANT, ARTICLE ABSORBANT, ET PROCÉDÉ DE TRAITEMENT DE SÉPARATION DE PARTICULES DE RÉSINE ABSORBANTE À PARTIR DE CET ARTICLE ABSORBANT

(30) Priority: 29.10.2021 JP 2021178144
(43) Date of publication of application: 04.09.2024
(73) Proprietor: Sumitomo Seika Chemicals Co., Ltd., Kako-gun, Hyogo 675-0145 (JP)
(72) Inventor: YAMAMOTO, Tomoe, Himeji-shi, Hyogo 672-8076 (JP)
(74) Representative: Mewburn Ellis LLP
(86) International application number: PCT/JP2022/040442
(87) International publication number: WO 2023/074861

(56) References cited:
- CN-A- 112 011 006
- CN-A- 112 011 006
- CN-A- 113 354 767
- CN-A- 113 354 767
- JP-A- 2002 285 021
- JP-A- 2005 097 604
- JP-A- 2012 183 175
- JP-A- H11 241 030
- JP-A- H11 241 030
- US-A1- 2003 004 479
- US-A1- 2005 113 252

## Description

### TECHNICAL FIELD

The present invention relates to a water-absorbing resin composition, an absorber, and an absorbent article, and more particularly to a water-absorbing resin composition constituting an absorber suitably used in hygienic materials such as disposable diapers, sanitary napkins, and incontinence pads, an absorber containing the water-absorbing resin composition, an absorbent article, and a method for separation treatment of water-absorbing resin particles from an absorbent article.

### BACKGROUND ART

In recent years, water-absorbing resins have been widely used in the field of hygienic materials such as disposable diapers, sanitary napkins, and incontinence pads.

As such a water-absorbing resin, a crosslinked product of a polymer of a partially neutralized acrylic acid salt has excellent water absorbing capacity, and acrylic acid as a raw material thereof is easily industrially available, thus the crosslinked product of the polymer can be produced at a constant quality and at a low cost. Also, since the crosslinked product of the polymer has many advantages such as being less likely to cause decomposition or deterioration, it is considered to be a preferable water-absorbing resin.

An absorbent article such as a disposable diaper, a sanitary napkin, or an incontinence pad mainly includes an absorber that is disposed in a central portion and absorbs and holds body fluids excreted from a body such as urine and menstrual blood, a liquid-permeable surface sheet (top sheet) disposed on a side coming in contact with the body, and a liquid-impermeable back sheet (back sheet) disposed on a side opposite to the side coming in contact with the body. The absorber is usually composed of hydrophilic fibers such as pulp and a water-absorbing resin.

Patent Documents 2 to 6 describe water-absorbing resin compositions comprising a water-absorbing resin and activated carbon.

### PRIOR ART DOCUMENT

### PATENT DOCUMENT

Patent Document 1: JP 6218982 B
Patent Document 2: US 2005/113252 A1
Patent Document 3: CN 112011006 A
Patent Document 4: JP H11-241030 A
Patent Document 5: US 2003/004479 A1
Patent Document 6: CN 113354767 A

### SUMMARY OF THE INVENTION

### PROBLEMS TO BE SOLVED BY THE INVENTION

It is a main object of the present invention to provide a water-absorbing resin composition containing water-absorbing resin particles and activated carbon, in which a gel that has absorbed water exhibits high stability in an environment such as normal use (wearing) of an absorbent article, and the gel can be adjusted to have a strength suitable for waste treatment (gel separating operation) by high-temperature treatment after use.

### MEANS FOR SOLVING THE PROBLEM

The present inventor has conducted intensive studies in order to solve the above problems. As a result, the present inventor has found that in a water-absorbing resin composition containing water-absorbing resin particles and activated carbon disposed on the surfaces of the water-absorbing resin particles, by using water-absorbing resin particles that have been subjected to a surface-crosslinking treatment and whose physiological saline absorption amount is set within a predetermined range as the water-absorbing resin particles, when the water-absorbing resin composition is used in an absorbent article, unexpectedly, a gel of the water-absorbing resin composition that has absorbed water is stable in a normal use environment of the absorbent article, and after use of the absorbent article, the gel strength can be adjusted to a strength suitable for waste treatment by treatment under high-temperature conditions for a long time. The present invention has been completed through further intensive studies with respect to such findings.

That is, the present invention provides an invention having the following configuration.
Item 1. A water-absorbing resin composition containing water-absorbing resin particles, and activated carbon disposed on surfaces of the water-absorbing resin particles,
   in which the water-absorbing resin particles are water-absorbing resin particles that have been subjected to a surface-crosslinking treatment,
   a physiological saline absorption amount of the water-absorbing resin particles is 30 to 80 g/g, and
   the activated carbon has a median particle size, which is measured by the method described in the description, of 1 µm or more and 70 µm or less.
Item 2. The water-absorbing resin composition according to item 1, in which a content of the activated carbon is 0.05 parts by mass or more and 1.0 part by mass or less with respect to 100 parts by mass of the water-absorbing resin particles.
Item 3. The water-absorbing resin composition according to item 1 or 2, in which the activated carbon has a median particle size of 1 µm or more and 45 µm or less.
Item 4. An absorber including the water-absorbing resin composition according to any one of items 1 to 3.
Item 5. An absorbent article including the absorber according to item 4.
Item 6. A method for separation treatment of a water-absorbing resin from the absorbent article according to item 5, the method including the step of heat treatment for 23 hours or more in an environment at a temperature of 70°C or higher and a relative humidity of 40% or higher.

### ADVANTAGES OF THE INVENTION

The present invention can provide a water-absorbing resin composition containing water-absorbing resin particles and activated carbon, in which when the water-absorbing resin composition is used in an absorbent article, a gel of the water-absorbing resin composition that has absorbed water is stable in a normal use environment of the absorbent article, and after use of the absorbent article, the gel strength can be adjusted to a strength suitable for waste treatment by treatment under high-temperature conditions for a long time. Furthermore, the present invention can also provide an absorber and an absorbent article using the water-absorbing resin composition.

### BRIEF DESCRIPTION OF THE DRAWINGS

Fig. 1 is a schematic view of an apparatus for measuring a physiological saline absorption amount under a load of 4.14 kPa.
Fig. 2 is a schematic view of a gel strength measuring apparatus.

### EMBODIMENTS OF THE INVENTION

In the present description, the term "comprising" includes "consisting essentially of" and "consisting of". In addition, in the present description, "(meth)acrylic" means" acrylic or methacrylic", and "(meth)acrylate" means" acrylate or methacrylate". The term "water-soluble" means that the solubility in water at 25°C is 5 mass% or more.

In addition, in the present description, a numerical value joined by "to" means a numerical range including numerical values before and after "to" as a lower limit value and an upper limit value. When a plurality of lower limit values and a plurality of upper limit values are described separately, any lower limit value and upper limit value can be selected and connected by "to".

### 1. Water-Absorbing Resin Composition

A water-absorbing resin composition of the present invention contains water-absorbing resin particles and activated carbon disposed on the surfaces of the water-absorbing resin particles, in which the water-absorbing resin particles are water-absorbing resin particles that have been subjected to a surface-crosslinking treatment, and a physiological saline absorption amount of the water-absorbing resin particles is 30 to 80 g/g. In the water-absorbing resin composition of the present invention having such characteristics, a gel of the water-absorbing resin composition that has absorbed water is stable in a normal use environment of the absorbent article, and after use of the absorbent article, the gel strength can be adjusted to a strength suitable for waste treatment by treatment under high-temperature conditions for a long time. Hereinafter, the water-absorbing resin composition of the present invention will be described in detail.

In the water-absorbing resin composition of the present invention, the "initial value of gel strength" evaluated by the method described in the examples is preferably 3700 N/m² or more, more preferably 3800 N/m² or more, still more preferably 4000 N/m² or more. The upper limit of the initial value of the gel strength is, for example, 6000 N/m².

In the water-absorbing resin composition of the present invention, the "gel strength after standing at 40°C for 14 hours" evaluated by the method described in the examples is preferably 3700 N/m² or more, more preferably 3800 N/m² or more, and still more preferably 4000 N/m² or more, from the viewpoint of stability of the absorbent article in a normal use environment. The upper limit of the gel strength is, for example, 6000 N/m².

In the water-absorbing resin composition of the present invention, the "gel strength after standing at 70°C for 24 hours" evaluated by the method described in the examples is preferably 3600 N/m² or less, more preferably 3500 N/m² or less, and still more preferably 3100 N/m² or less, from the viewpoint that the waste treatment is suitably performed.

### (Activated Carbon)

From the viewpoint of more suitably exerting the effect of the present invention, the median particle size of the activated carbon is 1 µm or more, preferably 10 µm or more, and more preferably 20 µm or more, and is 70 µm or less, and preferably 45 µm or less and 30 µm or less, and examples of the preferable range include 1 to 70 µm and 10 to 70 µm.

The median particle size (D 50 (median diameter), volume-based) of the activated carbon can be measured using a laser diffraction particle size distribution analyzer, and specifically, is a value measured by the method described in the examples.

From the viewpoint of more suitably exerting the effect of the present invention, the shape of the activated carbon is preferably a crushed shape, a columnar shape, or the like, and more preferably a crushed shape.

Further, from the viewpoint of more suitably exerting the effect of the present invention, the BET specific surface area of the activated carbon is preferably 100 m²/g or more, more preferably 1000 m²/g or more, and is preferably 3000 m²/g or less, more preferably 2000 m²/g or less, and examples of the preferable range include 100 to 3000 m²/g and 1000 to 2000 m²/g.

The BET specific surface area of activated carbon can be measured using a specific surface area analyzer, and specifically, is a value measured by the method described in the examples.

From the viewpoint of more suitably exerting the effect of the present invention, the activated carbon is preferably activated carbon having a polar functional group (hydrophilic functional group) on the surface (that is, hydrophilic activated carbon). Examples of the polar functional group include a hydroxy group, a carboxy group, and a phenol group. The activated carbon having a polar functional group on the surface is, for example, commercially available as activated carbon for a liquid phase, activated carbon for water treatment, and the like.

Examples of the origin of the activated carbon include coconut shells, infusible or carbonized organic materials, and infusible resins such as phenol resins. Examples of the organic material include polyacrylonitrile, pitch, polyvinyl alcohol, and cellulose. Among them, the origin of the activated carbon is preferably coconut shell or pitch (for example, coal pitch or cellulose pitch).

From the viewpoint of more suitably exerting the effect of the present invention, the content of activated carbon in the water-absorbing resin composition of the present invention is preferably 0.05 parts by mass or more, more preferably 0.1 parts by mass or more, still more preferably 0.2 parts by mass or more, and is preferably 1.0 part by mass or less, more preferably 0.5 parts by mass or less, still more preferably 0.4 parts by mass or less, and examples of the preferable range include 0.05 to 1.0 part by mass and 0.1 to 0.5 parts by mass with respect to 100 parts by mass of the water-absorbing resin particles.

From the viewpoint of more suitably exerting the effect of the present invention, the iodine adsorption amount of the activated carbon is preferably 100 mg/g or more, more preferably 500 mg/g or more, and is preferably 3000 mg/g or less, more preferably 2000 mg/g or less, and examples of the preferable range include 100 to 3000 mg/g and 500 to 2000 mg/g.

Here, the iodine adsorption amount of activated carbon is a value measured in accordance with JIS K1474: 2014.

From the viewpoint of more suitably exerting the effect of the present invention, the loss on drying of activated carbon is preferably 0.1% or more, more preferably 0.5% or more, and is preferably 5% or less, more preferably 3% or less, and examples of the preferable range include 0.1 to 5% and 0.5 to 3%.

Here, the loss on drying of activated carbon is a value measured in accordance with JIS K1474: 2014.

From the viewpoint of more suitably exerting the effect of the present invention, the pH of activated carbon is preferably 3 or more, more preferably 4 or more or 5 or more, and is preferably 11 or less, more preferably 9 or less, and examples of the preferable range include 3 to 11, 4 to 9, and 5 to 9.

Here, the pH of activated carbon is a value measured in accordance with JIS K1474: 2014.

In the water-absorbing resin composition of the present invention, the activated carbon is preferably disposed on the surfaces of the water-absorbing resin particles (that is, the activated carbon is present on the surfaces of the water-absorbing resin particles). As described later, for example, by mixing the water-absorbing resin particles and the activated carbon in a solid phase state, the activated carbon adheres to the surfaces of the water-absorbing resin particles, and the activated carbon can be disposed on the surfaces of the water-absorbing resin particles.

Next, the water-absorbing resin particles contained in the water-absorbing resin composition of the present invention will be described in detail.

### (Water-Absorbing Resin Particles)

The water-absorbing resin particles contained in the water-absorbing resin composition of the present invention is composed of a crosslinked polymer obtained by crosslinking a polymer of a water-soluble ethylenically unsaturated monomer, that is, a crosslinked polymer having a structural unit derived from a water-soluble ethylenically unsaturated monomer.

The water-absorbing resin particles of the present invention have been subjected to a surface-crosslinking treatment and are surface-crosslinked water-absorbing resin particles. In the present invention, the physiological saline absorption amount of the water-absorbing resin particles is 30 to 80 g/g. In the water-absorbing resin composition of the present invention, by using such specific water-absorbing resin particles together with activated carbon, when the water-absorbing resin composition is used in an absorbent article, a gel of the water-absorbing resin composition that has absorbed water is stable in a normal use environment of the absorbent article. After use of the absorbent article, the gel strength can be adjusted to a strength suitable for waste treatment by treatment under high-temperature conditions for a long time. A specific method of the surface-crosslinking treatment of the surface-crosslinked water-absorbing resin particles used in the present invention will be described in <Surface-Crosslinking Step> below.

From the viewpoint of more suitably exerting the effect of the present invention, the physiological saline absorption amount of the water-absorbing resin particles is 30 g/g or more, preferably 50 g/g or more, more preferably 60 g/g or more, and is 80 g/g or less, preferably 70 g/g or less, more preferably 65 g/g or less.

In addition, from the viewpoint of more suitably exerting the effect of the present invention, the physiological saline absorption amount under a load of 4.14 kPa of the water-absorbing resin particles is preferably 10 ml/g or more, more preferably 13 ml/g or more, still more preferably 15 ml/g or more, and is preferably 40 ml/g or less, more preferably 35 ml/g or less, still more preferably 30 ml/g or less, and examples of the preferable range include 15 to 30 ml/g.

Each of the physiological saline retention amount and the physiological saline absorption amount under a load of 4.14 kPa of the water-absorbing resin particles is a value measured by the method described in the examples.

Further, from the viewpoint of more suitably exerting the effect of the present invention, the BET specific surface area of the water-absorbing resin particles is preferably 0.01 m²/g or more, more preferably 0.02 m²/g or more, and is preferably 0.150 m²/g or less, more preferably 0.05 m²/g or less, and examples of the preferable range include 0.01 to 0.150 m²/g and 0.02 to 0.05 m²/g.

The BET specific surface area of the water-absorbing resin particles can be measured using a specific surface area analyzer, and specifically, the BET specific surface area is a value measured by the method described in the examples.

The water-absorbing resin is usually in particulate form. The median particle size of the water-absorbing resin particles is preferably 150 µm or more, 200 µm or more, 240 µm or more, 260 µm or more, 280 µm or more, or 300 µm or more from the viewpoint of suitably exerting the effect of the present invention while avoiding local absorption in the absorbent article. In addition, the median particle size is preferably 850 µm or less, 600 µm or less, 550 µm or less, 500 µm or less, 450 µm or less, or 400 µm or less from the viewpoint of suitably exerting the effect of the present invention while making the tactile sensation of the absorbent article comfortable. That is, the median particle size is preferably 150 to 850 µm, more preferably 200 to 600 µm, still more preferably 240 to 500 µm, even still more preferably 280 to 450 µm, and further preferably 300 to 400 µm. Also in the water-absorbing resin composition of the present invention, the median particle size is preferably 150 to 850 µm, more preferably 200 to 600 µm, still more preferably 240 to 500 µm, even still more preferably 280 to 450 µm, and further more preferably 300 to 400 µm.

Other than a form in which each of the water-absorbing resin particles is composed of a single particle, the water-absorbing resin particles may be in a form (secondary particles) in which fine particles (primary particles) are aggregated. Examples of the shape of the primary particle include a substantially spherical shape, an indefinite crushed shape, and a plate shape. Examples of the primary particles produced by reversed-phase suspension polymerization include substantially spherical single particles having a smooth surface shape such as a perfect spherical shape or an elliptical spherical shape.

The median particle size of the water-absorbing resin particles can be measured using JIS standard sieves, and specifically, is a value measured by the method described in the examples.

As a polymerization method of the water-soluble ethylenically unsaturated monomer, one of an aqueous solution polymerization method, an emulsion polymerization method, a reversed-phase suspension polymerization method, and the like, which are representative polymerization methods, is used. In the aqueous solution polymerization method, polymerization is performed by heating an aqueous solution of a water-soluble ethylenically unsaturated monomer while stirring the aqueous solution as necessary. In the reversed-phase suspension polymerization method, polymerization is performed by heating a water-soluble ethylenically unsaturated monomer in a hydrocarbon dispersion medium under stirring.

An example of the method for producing the water-absorbing resin particles will be described below.

Specific examples of the method for producing water-absorbing resin particles include a method for producing water-absorbing resin particles by performing reversed-phase suspension polymerization of a water-soluble ethylenically unsaturated monomer in a hydrocarbon dispersion medium, the method including a step of performing polymerization in the presence of a radical polymerization initiator and a step of surface-crosslinking a hydrous gel obtained by the polymerization in the presence of a surface-crosslinking agent. **In** the method for producing water-absorbing resin particles of the present invention, an internal-crosslinking agent may be added to the water-soluble ethylenically unsaturated monomer as necessary to form a hydrous gel having an internally crosslinked structure.

### <Polymerization Step>

### [Water-Soluble Ethylenically Unsaturated Monomer]

Examples of the water-soluble ethylenically unsaturated monomer include (meth)acrylic acid (in the present description, "acrylic" and "methacrylic" are collectively referred to as "(meth)acrylic", and the same applies hereinafter) and salts thereof; 2-(meth)acrylamide-2-methylpropanesulfonic acid and salts thereof; nonionic monomers such as (meth)acrylamide, N,N-dimethyl (meth)acrylamide, 2-hydroxyethyl (meth)acrylate, N-methylol (meth)acrylamide, and polyethylene glycol mono(meth)acrylate; amino group-containing unsaturated monomers such as N,N-diethylaminoethyl (meth)acrylate, N,N-diethylaminopropyl (meth)acrylate, and diethylaminopropyl (meth)acrylamide, and quaternized products thereof. Among these water-soluble ethylenically unsaturated monomers, from the viewpoints of industrial availability and the like, (meth)acrylic acid or salts thereof, (meth)acrylamide, and N,N-dimethylacrylamide are preferable, and (meth)acrylic acid and salts thereof are more preferable. These water-soluble ethylenically unsaturated monomers may be used singly or in combination of two or more.

Among them, acrylic acid and salts thereof are widely used as raw materials of the water-absorbing resin particles, and the acrylic acid and/or a salt thereof may be copolymerized with the above-described other water-soluble ethylenically unsaturated monomer and then used. In this case, the acrylic acid and/or a salt thereof is preferably used as a main water-soluble ethylenically unsaturated monomer in an amount of 70 to 100 mol% with respect to the total amount of water-soluble ethylenically unsaturated monomers.

The water-soluble ethylenically unsaturated monomer may be dispersed in a state of an aqueous solution in a hydrocarbon dispersion medium and subjected to reversed-phase suspension polymerization. When the water-soluble ethylenically unsaturated monomer is an aqueous solution, the dispersion efficiency in the hydrocarbon dispersion medium can be increased. The concentration of the water-soluble ethylenically unsaturated monomer in this aqueous solution is preferably in a range of 20 mass% to a saturated concentration or less. The concentration of the water-soluble ethylenically unsaturated monomer is more preferably 55 mass% or less, still more preferably 50 mass% or less, and even still more preferably 45 mass% or less. On the other hand, the concentration of the water-soluble ethylenically unsaturated monomer is more preferably 25 mass% or more, still more preferably 28 mass% or more, and even still more preferably 30 mass% or more.

Like (meth)acrylic acid, 2-(meth)acrylamide-2-methylpropanesulfonic acid, or similar monomer, when the water-soluble ethylenically unsaturated monomer has an acid group, the acid group may be neutralized with an alkaline neutralizing agent as necessary prior to use. Examples of such an alkaline neutralizing agent include alkali metal salts such as sodium hydroxide, sodium carbonate, sodium hydrogen carbonate, potassium hydroxide, and potassium carbonate; and ammonia. In addition, these alkaline neutralizing agents may be used in a form of an aqueous solution in order to simplify the neutralization operation. The alkaline neutralizing agents described above may be used singly or in combination of two or more.

The degree of neutralization of the water-soluble ethylenically unsaturated monomer by the alkaline neutralizing agent is preferably 10 to 100 mol%, more preferably 30 to 90 mol%, still more preferably 40 to 85 mol%, and even still more preferably 50 to 80 mol% as the degree of neutralization with respect to all the acid groups of the water-soluble ethylenically unsaturated monomer.

### [Radical Polymerization Initiator]

Examples of the radical polymerization initiator added to the polymerization step include persulfates such as potassium persulfate, ammonium persulfate, and sodium persulfate, peroxides such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide, and azo compounds such as 2,2'-azobis(2-amidinopropane) dihydrochloride, 2,2'-azobis[2-(N-phenylamidino)propane] dihydrochloride, 2,2'-azobis[2-(N-allylamidino)propane] dihydrochloride, 2,2'-azobis{2-[1-(2-hydroxyethyl)-2-imidazolin-2-yl]propane} dihydrochloride, 2,2'-azobis{2-methyl-N-[1,1-bis(hydroxymethyl)-2-hydroxyethyl]propionamide}, 2,2'-azobis[2-methyl-N-(2-hydroxyethyl)-propionamide], and 4,4'-azobis(4-cyanovaleric acid). Among these radical polymerization initiators, potassium persulfate, ammonium persulfate, sodium persulfate, and 2,2'-azobis(2-amidinopropane) dihydrochloride are preferable from the viewpoints of easy availability and easy handling. These radical polymerization initiators may be used singly or in combination of two or more. The radical polymerization initiator can also be used as a redox polymerization initiator in combination with a reducing agent such as sodium sulfite, sodium hydrogen sulfite, ferrous sulfate, or L-ascorbic acid.

The amount of the radical polymerization initiator used is, for example, 0.00005 to 0.01 mol with respect to 1 mol of the water-soluble ethylenically unsaturated monomer. By satisfying such an amount used, occurrence of a rapid polymerization reaction can be avoided, and the polymerization reaction can be completed in an appropriate time.

### [Internal-Crosslinking Agent]

Examples of the internal-crosslinking agent include those capable of crosslinking a polymer of a water-soluble ethylenically unsaturated monomer to be used, and examples thereof include (poly)ethylene glycol ["(poly)" means the case where there is or is not a prefix "poly", and the same applies hereinafter], unsaturated polyesters obtained by reacting polyols including diols and triols such as (poly)propylene glycol, 1,4-butanediol, 1,6-hexanediol, trimethylolpropane, and (poly)glycerin with unsaturated acids such as (meth)acrylic acid, maleic acid, and fumaric acid; bisacrylamides such as N,N-methylenebisacrylamide; di(meth)acrylic acid esters or tri(meth)acrylic acid esters obtained by reacting polyepoxide with (meth)acrylic acid; di(meth)acrylic acid carbamyl esters obtained by reacting polyisocyanates such as tolylene diisocyanate and hexamethylene diisocyanate with hydroxyethyl (meth)acrylate; compounds having two or more polymerizable unsaturated groups, such as allylated starch, allylated cellulose, diallyl phthalate, N,N',N"-triallyl isocyanurate, and divinylbenzene; polyglycidyl compounds including diglycidyl compounds such as (poly)ethylene glycol diglycidyl ether, (poly)propylene glycol diglycidyl ether, and (poly)glycerin diglycidyl ether and triglycidyl compounds; epihalohydrin compounds such as epichlorhydrin, epibromhydrin, and α-methylepichlorhydrin; compounds having two or more reactive functional groups including isocyanate compounds such as 2,4-tolylene diisocyanate and hexamethylene diisocyanate; and oxetane compounds such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol. Among these internal-crosslinking agents, polyglycidyl compounds are preferably used, diglycidyl ether compounds are more preferably used, and (poly)ethylene glycol diglycidyl ether, (poly)propylene glycol diglycidyl ether, and (poly)glycerin diglycidyl ether are preferably used. These internal-crosslinking agents may be used singly or in combination of two or more.

The amount of the internal-crosslinking agent used is preferably 0.000001 to 0.02 mol, more preferably 0.00001 to 0.01 mol, still more preferably 0.00001 to 0.005 mol, and even still more preferably 0.00005 to 0.002 mol with respect to 1 mol of the water-soluble ethylenically unsaturated monomer.

### [Hydrocarbon Dispersion Medium]

Examples of the hydrocarbon dispersion medium include aliphatic hydrocarbons having 6 to 8 carbon atoms such as n-hexane, n-heptane, 2-methylhexane, 3-methylhexane, 2,3-dimethylpentane, 3-ethylpentane, and n-octane; alicyclic hydrocarbons such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons such as benzene, toluene, and xylene. Among these hydrocarbon dispersion media, n-hexane, n-heptane, and cyclohexane are particularly suitably used because they are industrially easily available, have stable quality, and are inexpensive. These hydrocarbon dispersion media may be used singly or in combination of two or more. As an example of the mixture of the hydrocarbon dispersion media, a suitable result can be obtained also by using a commercially available product such as Exxsol Heptane (manufactured by Exxon Mobil Corporation: containing 75 to 85 mass% hydrocarbons of heptane and an isomer thereof).

The amount of the hydrocarbon dispersion medium used is preferably 100 to 1500 parts by mass, and more preferably 200 to 1400 parts by mass with respect to 100 parts by mass of the first-stage water-soluble ethylenically unsaturated monomer from the viewpoints of uniformly dispersing the water-soluble ethylenically unsaturated monomer and easily controlling the polymerization temperature. As will be described later, the reversed-phase suspension polymerization is performed in one stage (single stage) or two or more stages, and the above-described first-stage polymerization means the first-stage polymerization reaction in the single-stage polymerization or the multistage polymerization (the same applies hereinafter).

### [Dispersion Stabilizer]

### (Surfactant)

In the reversed-phase suspension polymerization, a dispersion stabilizer can also be used in order to improve the dispersion stability of the water-soluble ethylenically unsaturated monomer in the hydrocarbon dispersion medium. As the dispersion stabilizer, a surfactant can be used.

As the surfactant, for example, a sucrose fatty acid ester, a polyglycerin fatty acid ester, a sorbitan fatty acid ester, a polyoxyethylene sorbitan fatty acid ester, a polyoxyethylene glycerin fatty acid ester, a sorbitol fatty acid ester, a polyoxyethylene sorbitol fatty acid ester, a polyoxyethylene alkyl ether, a polyoxyethylene alkyl phenyl ether, a polyoxyethylene castor oil, a polyoxyethylene hydrogenated castor oil, an alkylallyl formaldehyde condensed polyoxyethylene ether, a polyoxyethylene polyoxypropylene block copolymer, a polyoxyethylene polyoxypropyl alkyl ether, a polyethylene glycol fatty acid ester, an alkyl glucoside, an N-alkyl gluconamide, a polyoxyethylene fatty acid amide, a polyoxyethylene alkylamine, a phosphoric acid ester of a polyoxyethylene alkyl ether, a phosphoric acid ester of a polyoxyethylene alkyl allyl ether, or the like can be used. Among these surfactants, it is particularly preferable to use a sorbitan fatty acid ester, a polyglycerin fatty acid ester, or a sucrose fatty acid ester from the viewpoint of dispersion stability of the monomer. These surfactants may be used singly or in combination of two or more.

The amount of the surfactant used is preferably 0.1 to 30 parts by mass, and more preferably 0.3 to 20 parts by mass with respect to 100 parts by mass of the first-stage water-soluble ethylenically unsaturated monomer.

### (Polymeric Dispersant)

As the dispersion stabilizer used in the reversed-phase suspension polymerization, a polymeric dispersant may be used together with the surfactant described above.

Examples of the polymeric dispersant include maleic anhydride-modified polyethylene, maleic anhydride-modified polypropylene, maleic anhydride-modified ethylene-propylene copolymer, maleic anhydride-modified ethylene-propylene-diene terpolymer (EPDM), maleic anhydride-modified polybutadiene, maleic anhydride-ethylene copolymer, maleic anhydride-propylene copolymer, maleic anhydride-ethylene-propylene copolymer, maleic anhydride-butadiene copolymer, polyethylene, polypropylene, ethylene-propylene copolymer, oxidized polyethylene, oxidized polypropylene, oxidized ethylene-propylene copolymer, ethylene-acrylic acid copolymer, ethyl cellulose, and ethyl hydroxyethyl cellulose. Among these polymeric dispersants, maleic anhydride-modified polyethylene, maleic anhydride-modified polypropylene, maleic anhydride-modified ethylene-propylene copolymer, maleic anhydride-ethylene copolymer, maleic anhydride-propylene copolymer, maleic anhydride-ethylene-propylene copolymer, polyethylene, polypropylene, ethylene-propylene copolymer, oxidized polyethylene, oxidized polypropylene, and oxidized ethylene-propylene copolymer are particularly preferably used from the viewpoint of dispersion stability of monomers. These polymeric dispersants may be used singly or in combination of two or more.

The amount of the polymeric dispersant used is preferably 0.1 to 30 parts by mass, and more preferably 0.3 to 20 parts by mass with respect to 100 parts by mass of the first-stage water-soluble ethylenically unsaturated monomer.

### [Other Components]

In the method for producing water-absorbing resin particles, if desired, other components may be added to an aqueous solution containing the water-soluble ethylenically unsaturated monomer, and then the reversed-phase suspension polymerization may be performed. As other components, various additives such as a thickener and a chain transfer agent can be added.

As an example, reversed-phase suspension polymerization can be performed after adding a thickener to an aqueous solution containing the water-soluble ethylenically unsaturated monomer. By thus adding a thickener to adjust the viscosity of the aqueous solution, it is possible to control the median particle size obtained in the reversed-phase suspension polymerization.

As the thickener, for example, hydroxyethyl cellulose, hydroxypropyl cellulose, methyl cellulose, carboxymethyl cellulose, polyacrylic acid, a (partial) neutralized polyacrylic acid, polyethylene glycol, polyacrylamide, polyethyleneimine, dextrin, sodium alginate, polyvinyl alcohol, polyvinylpyrrolidone, polyethylene oxide, or the like can be used. When the stirring speed during polymerization is the same, the primary particles and/or the secondary particles of the obtained particles tend to be larger as the viscosity of the aqueous solution of the water-soluble ethylenically unsaturated monomer is higher.

### [Reversed-Phase Suspension Polymerization]

In performing the reversed-phase suspension polymerization, for example, an aqueous monomer solution containing a water-soluble ethylenically unsaturated monomer is dispersed in a hydrocarbon dispersion medium in the presence of a dispersion stabilizer. In this case, as long as it is before start of the polymerization reaction, the addition timing of the dispersion stabilizer (surfactant or polymeric dispersant) may be either before or after the addition of the aqueous monomer solution.

Among them, from the viewpoint of easily reducing the amount of the hydrocarbon dispersion medium remaining in the resulting water-absorbing resin particles, it is preferable to disperse the aqueous monomer solution in the hydrocarbon dispersion medium in which the polymeric dispersant has been dispersed, and then to further disperse the surfactant therein before performing polymerization.

Such reversed-phase suspension polymerization can be performed in one stage or two or more stages. In addition, from the viewpoint of enhancing productivity, it is preferable to perform 2 to 3 stages.

When the reversed-phase suspension polymerization is performed in two or more stages, the first-stage reversed-phase suspension polymerization is performed, then the water-soluble ethylenically unsaturated monomer is added to and mixed with the reaction mixture obtained in the first-stage polymerization reaction, and the second-stage or subsequent reversed-phase suspension polymerization may be performed in the same manner as the first-stage polymerization. In the reversed-phase suspension polymerization in each of the second and subsequent stages, in addition to the water-soluble ethylenically unsaturated monomer, a radical polymerization initiator is preferably added within the range of the molar ratio of each component to the water-soluble ethylenically unsaturated monomer described above based on the amount of the water-soluble ethylenically unsaturated monomer added in the reversed-phase suspension polymerization in each of the second and subsequent stages, to perform reversed-phase suspension polymerization. In the second and subsequent polymerization, an internal-crosslinking agent may be added to the water-soluble ethylenically unsaturated monomer as necessary.

The reaction temperature of the polymerization reaction is preferably 20 to 110°C and more preferably 40 to 90°C from the viewpoints of enhancing the economic efficiency by allowing the polymerization to proceed rapidly and shortening the polymerization time, and allowing the reaction to proceed smoothly by easily removing the heat of polymerization.

### <Surface-Crosslinking Step>

Next, the water-absorbing resin particles of the present invention are obtained by adding a surface-crosslinking agent to the hydrous gel having an internally crosslinked structure that has been obtained by polymerizing the water-soluble ethylenically unsaturated monomer, and crosslinking the hydrous gel (surface-crosslinking reaction). This surface-crosslinking reaction is preferably performed in the presence of a surface-crosslinking agent after polymerization of the water-soluble ethylenically unsaturated monomer. As described above, by subjecting the hydrous gel having an internally crosslinked structure to a surface-crosslinking reaction after polymerization, it is possible to obtain water-absorbing resin particles in which the crosslinking density in the vicinity of the surface of the water-absorbing resin particle is increased and various performances such as water absorption capacity under load are improved.

Examples of the surface-crosslinking agent include compounds having two or more reactive functional groups. Examples include polyols such as ethylene glycol, propylene glycol, 1,4-butanediol, diethylene glycol, triethylene glycol, trimethylolpropane, glycerin, polyoxyethylene glycol, polyoxypropylene glycol, and polyglycerin; polyglycidyl compounds such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether; haloepoxy compounds such as epichlorhydrin, epibromhydrin, and α-methylepichlorhydrin; isocyanate compounds such as 2,4-tolylene diisocyanate and hexamethylene diisocyanate; oxetane compounds such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol; oxazoline compounds such as 1,2-ethylene bisoxazoline; carbonate compounds (for example, alkylene carbonate) such as ethylene carbonate, propylene carbonate, 4,5-dimethyl-1,3-dioxolan-2-one, 4,4-dimethyl-1,3-dioxolan-2-one, 4-ethyl-1,3-dioxolan-2-one, 4-hydroxymethyl-1,3-dioxolan-2-one, 1,3-dioxan-2-one, 4-methyl-1,3-dioxan-2-one, 4,6-dimethyl-1,3-dioxan-2-one, and 1,3-dioxolan-2-one; and hydroxyalkylamide compounds such as bis[N,N-di(β-hydroxyethyl)]adipamide. Among these surface-crosslinking agents, polyglycidyl compounds such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether are preferable. These surface-crosslinking agents may be used singly or in combination of two or more.

The amount of the surface-crosslinking agent used is preferably 0.00001 to 0.01 mol, more preferably 0.00005 to 0.005 mol, and still more preferably 0.0001 to 0.002 mol with respect to 1 mol of the total amount of the water-soluble ethylenically unsaturated monomers used for polymerization.

As a method for adding the surface-crosslinking agent, the surface-crosslinking agent may be added as it is or as an aqueous solution, or may be added as a solution using a hydrophilic organic solvent as a solvent as necessary. Examples of the hydrophilic organic solvent include lower alcohols such as methyl alcohol, ethyl alcohol, n-propyl alcohol, and isopropyl alcohol; ketones such as acetone and methyl ethyl ketone; ethers such as diethyl ether, dioxane, and tetrahydrofuran; amides such as N,N-dimethylformamide; and sulfoxides such as dimethyl sulfoxide. These hydrophilic organic solvents may be used singly or in combination of two or more, or as a mixed solvent obtained by mixing with water.

The surface-crosslinking agent may be added at any timing as long as it is added after almost all the polymerization reaction of the water-soluble ethylenically unsaturated monomer is completed. The surface-crosslinking agent is preferably added in the presence of moisture in a range of 1 to 400 parts by mass, more preferably in the presence of moisture in a range of 5 to 200 parts by mass, still more preferably in the presence of moisture in a range of 10 to 100 parts by mass, and even still more preferably in the presence of moisture in a range of 20 to 60 parts by mass with respect to 100 parts by mass of the water-soluble ethylenically unsaturated monomer. The amount of moisture means the total amount of moisture contained in the reaction system and moisture used as necessary when the surface-crosslinking agent is added.

The reaction temperature in the surface-crosslinking reaction is preferably 50 to 250°C, more preferably 60 to 180°C, still more preferably 60 to 140°C, and even still more preferably 70 to 120°C. The reaction time of the surface-crosslinking reaction is preferably 1 to 300 minutes, and more preferably 5 to 200 minutes.

### <Drying Step>

The method may include a drying step of removing water, a hydrocarbon dispersion medium, and the like by externally applying energy such as heat and carrying out distillation after performing the reversed-phase suspension polymerization described above. When dehydration of the hydrous gel after the reversed-phase suspension polymerization is performed, the system in which the hydrous gel is dispersed in the hydrocarbon dispersion medium is heated, so that water and the hydrocarbon dispersion medium are temporarily distilled off to the outside of the system by azeotropic distillation. At this time, when only the distilled hydrocarbon dispersion medium is returned into the system, continuous azeotropic distillation becomes possible. Since the temperature in the system during drying is maintained at an azeotropic temperature with the hydrocarbon dispersion medium or lower in that case, it is preferable from the viewpoint that the resin is less likely to deteriorate. Subsequently, water and the hydrocarbon dispersion medium are distilled off to obtain the water-absorbing resin particles. Various performances of the water-absorbing resin particles to be obtained can be controlled by controlling the treatment conditions in the drying step after the polymerization to adjust the amount of water to be removed.

In the drying step, the drying treatment by distillation may be performed under normal pressure or under reduced pressure. From the viewpoint of enhancing the drying efficiency, the drying may be performed under a flow of nitrogen or the like. When the drying treatment is performed under normal pressure, the drying temperature is preferably 70 to 250°C, more preferably 80 to 180°C, still more preferably 80 to 140°C, and even still more preferably 90 to 130°C. When the drying treatment is performed under reduced pressure, the drying temperature is preferably 40 to 160°C, and more preferably 50 to 110°C.

When the surface-crosslinking step with the surface-crosslinking agent is performed after the polymerization of the monomer by reversed-phase suspension polymerization, the drying step by distillation described above is performed after the completion of the surface-crosslinking step. Alternatively, the surface-crosslinking step and the drying step may be performed simultaneously.

The water-absorbing resin composition of the present invention may contain an additive according to the purpose, in addition to the acidic compound. Examples of such an additive include inorganic powders, surfactants, oxidizing agents, reducing agents, metal chelating agents, radical chain inhibitors, antioxidants, and antibacterial agents. For example, the flowability of the water-absorbing resin composition can be further improved by adding 0.05 to 5 parts by mass of amorphous silica as an inorganic powder with respect to 100 parts by mass of the water-absorbing resin particles. The additive is preferably hydrophilic or water-soluble.

In the water-absorbing resin composition of the present invention, the content of the water-absorbing resin particles (excluding additives) is preferably 70 mass% or more, more preferably 80 mass% or more, and still more preferably 90 mass% or more.

The water-absorbing resin composition of the present invention can be produced, for example, by mixing the water-absorbing resin particles and the activated carbon in a solid phase state.

### 2. Absorber and Absorbent Article

The water-absorbing resin composition of the present invention constitutes an absorber used in, for example, hygienic materials such as sanitary products and disposable diapers, and is suitably used in an absorbent article including the absorber.

Here, the absorber using the water-absorbing resin composition of the present invention contains the particulate water-absorbing resin composition of the present invention. The absorber may further contain hydrophilic fibers. Examples of the configuration of the absorber include a sheet-like structure in which water-absorbing resin particles are fixed on a nonwoven fabric or between a plurality of nonwoven fabrics, a mixed dispersion obtained by mixing a particulate water-absorbing resin composition and hydrophilic fibers so as to have a uniform composition, a sandwich structure in which a particulate water-absorbing resin composition is sandwiched between layered hydrophilic fibers, and a structure in which a particulate water-absorbing resin composition and hydrophilic fibers are wrapped with tissue. The absorber may contain other components, for example, an adhesive binder such as a heat-fusible synthetic fiber, a hot melt adhesive, or an adhesive emulsion for enhancing the shape retention of the absorber.

The content of the water-absorbing resin composition in the absorber is preferably 5 to 100 mass%, more preferably 10 to 95 mass%, still more preferably 20 to 90 mass%, and even still more preferably 30 to 80 mass%.

Examples of the hydrophilic fibers include cellulose fibers obtained from wood such as fluff pulp, mechanical pulp, chemical pulp, and semi-chemical pulp, artificial cellulose fibers such as rayon and acetate, and hydrophilized fibers made of synthetic resins such as polyamide, polyester, and polyolefin. The average fiber length of the hydrophilic fibers is usually 0.1 to 10 mm or may be 0.5 to 5 mm.

The absorber using the particulate water-absorbing resin composition of the present invention can be held between a liquid-permeable sheet (top sheet) through which a liquid can pass and a liquid-impermeable sheet (back sheet) through which a liquid cannot pass to form the absorbent article of the present invention. The liquid-permeable sheet is disposed on the side coming in contact with a body, and the liquid-impermeable sheet is disposed on a side opposite to the side coming in contact with the body.

Examples of the liquid-permeable sheet include an air-through type, a spunbond type, a chemical bond type, a needle punch type, and similar type nonwoven fabrics made of fibers such as polyethylene, polypropylene, and polyester, and porous synthetic resin sheets. Examples of the liquid-impermeable sheet include synthetic resin films made of resins such as polyethylene, polypropylene, and polyvinyl chloride.

### 3. Method for Separation Treatment of Water-Absorbing Resin from Absorbent Article

The absorbent article of the present invention includes the water-absorbing resin composition of the present invention. As described above, when the water-absorbing resin composition of the present invention is used in an absorbent article, a gel of the water-absorbing resin composition that has absorbed water is stable in a normal use environment of the absorbent article, and after use of the absorbent article, the gel strength can be adjusted to a strength suitable for waste treatment by treatment under high-temperature conditions for a long time.

In the method for separation treatment of water-absorbing resin particles from an absorbent article of the present invention, a separation step of heat treatment for 20 hours or more in an environment at a temperature of 70°C or higher and a relative humidity of 40% or higher is performed. By performing this step, the gel strength of the water-absorbing resin particles contained in the absorbent article is adjusted to a strength suitable for waste treatment (that is, the gel strength is appropriately lowered, whereby the water-absorbing resin particles are adjusted to have a property of being easily separated from other materials constituting the absorbent article), and the water-absorbing resin can be suitably separated from the absorbent article.

The temperature in the separation step is preferably 70 to 120°C, more preferably 70 to 100°C, the relative humidity is 40 to 100%, preferably 40 to 90%, and the heat treatment time is 23 hours or more, preferably 30 hours or more.

### EXAMPLES

Hereinafter, the present invention will be described in detail with reference to the examples and the comparative example. However, the present invention is not limited to the examples.

The water-absorbing resin particles obtained in the following production example and the water-absorbing resin compositions obtained in the examples and the comparative example were evaluated in the following various tests. Unless otherwise specified, the measurement was performed under an environment of a temperature of 25 ± 2°C and a humidity of 50 ± 10%.

### [Production of Water-Absorbing Resin Particles]

### <Production Example 1>

A 2-L round-bottom cylindrical separable flask having an inner diameter of 11 cm and equipped with a reflux condenser, a dropping funnel, a nitrogen gas inlet tube, and a stirring blade having two stages of 4-inclined paddle blades having a blade diameter of 5 cm as a stirrer was prepared. In this flask, 293 g of n-heptane as a hydrocarbon dispersion medium was placed, 0.736 g of a maleic anhydride-modified ethylene-propylene copolymer (Mitsui Chemicals, Inc., Hi-WAX 1105A) as a polymeric dispersant was added thereto, the temperature was raised to 80°C with stirring to dissolve the dispersant, and then the contents were cooled to 50°C. In a beaker having an internal volume of 300 mL, 92.0 g (1.03 mol) of an 80.5 mass% aqueous acrylic acid solution as a water-soluble ethylenically unsaturated monomer was placed, 147.7 g of a 20.9 mass% aqueous sodium hydroxide solution was added dropwise while cooling with ice water to perform neutralization of 75 mol%, and then 0.092 g of hydroxylethyl cellulose (Sumitomo Seika Chemicals Co., Ltd., HEC AW-15F) as a thickener, 0.0736 g (0.272 mmol) of potassium persulfate as a water-soluble radical polymerization agent, and 0.010 g (0.057 mmol) of ethylene glycol diglycidyl ether as an internal-crosslinking agent were added thereto and dissolved, thereby preparing a first-stage aqueous solution. Then, the aqueous solution prepared above was added to the separable flask and stirred for 10 minutes, after which a surfactant solution obtained by heating and dissolving 0.736 g of a sucrose stearate having an HLB of 3 (Mitsubishi Chemical Foods Corporation, RYOTO Sugar Ester S-370) as a surfactant in 6.62 g of n-heptane in a 20 mL-vial was further added. While stirring at a stirrer rotational speed of 550 rpm, the inside of the system was sufficiently purged with nitrogen, and then the flask was heated by immersing in a water bath at 70°C, and polymerization was performed for 60 minutes to obtain a first-stage polymerization slurry solution.

In another beaker having an internal volume of 500 mL, 128.8 g (1.43 mol) of an 80.5 mass% aqueous acrylic acid solution as a water-soluble ethylenically unsaturated monomer was placed, 159.0 g of a 27 mass% aqueous sodium hydroxide solution was added dropwise while cooling with ice water to perform neutralization of 75 mol%, and then 0.103 g (0.381 mmol) of potassium persulfate as a water-soluble radical polymerization initiator and 0.0117 g (0.067 mmol) of ethylene glycol diglycidyl ether as an internal-crosslinking agent were added thereto and dissolved, thereby preparing a second-stage aqueous solution.

While stirring at a stirrer rotational speed of 1000 rpm, the inside of the separable flask system was cooled to 25°C. Then the whole amount of the second-stage aqueous solution was added to the first-stage polymerization slurry. After the inside of the system was purged with nitrogen for 30 minutes, the flask was heated by immersing in a water bath at 70°C again, and polymerization was performed for 60 minutes to obtain a hydrous gel polymer.

To the second-stage hydrous gel polymer, 0.491 g of a 45 mass% aqueous pentasodium diethylenetriaminepentaacetate solution was added under stirring. Thereafter, the flask was immersed in an oil bath set at 125°C, and 244.5 g of water was removed to the outside of the system while refluxing n-heptane by azeotropic distillation of n-heptane and water. Then, 4.42 g of a 2 mass% aqueous solution of ethylene glycol diglycidyl ether (0.507 mmol) as a surface-crosslinking agent was added to the flask, and the contents were held at 83°C for 2 hours.

Thereafter, n-heptane was evaporated at 125°C, dried, and further passed through a sieve with an aperture of 850 µm to obtain 222.9 g of water-absorbing resin particles. The physiological saline absorption amount of the water-absorbing resin particles was 63 g/g, the median particle size thereof was 346 µm, the physiological saline absorption amount under a load of 4.14 kPa thereof was 21 ml/g, and the BET specific surface area thereof was 0.033 m²/g.

### [Evaluation of Water-Absorbing Resin Particles]

### <Physiological Saline Absorption Amount>

A 0.9 mass% aqueous sodium chloride solution (physiological saline), 500 g, was weighed out in a 500 mL beaker, and 2.0 g of water-absorbing resin particles were dispersed therein while stirring at 600 rpm with a 3 cm stirrer bar (no ring) so as not to form lumps. The mixture was allowed to stand for 60 minutes with stirring to sufficiently swell the water-absorbing resin particles. Thereafter, the mass of a standard sieve having an aperture of 75 µm Wa (g) was measured in advance, and the contents in the beaker were filtered using the sieve, and the sieve was allowed to stand for 30 minutes in a state where the sieve was inclined at an inclination angle of about 30 degrees with respect to the horizontal, thereby filtering out excess moisture. The mass of the sieve containing the swollen gel Wb (g) was measured, and the physiological saline absorption capacity was calculated by the following formula. $Physiological saline absorption amount \left(g/g\right) = \left[Wb-Wa\right] / 2.0$

### <Median Particle Size (Particle Size Distribution)>

Water-absorbing resin particles, 50 g, were used for measuring the median particle size (particle size distribution). JIS standard sieves were combined in the following order from the top: a sieve with an aperture of 850 µm, a sieve with an aperture of 500 µm, a sieve with an aperture of 425 µm, a sieve with an aperture of 300 µm, a sieve with an aperture of 250 µm, a sieve with an aperture of 180 µm, a sieve with an aperture of 150 µm, and a receptacle. The water-absorbing resin particles were put into the uppermost sieve of the combined sieves, and shaken for 20 minutes using a rotating and tapping shaker to perform classification. After classification, the mass of the water-absorbing resin particles retained on each sieve was calculated as a mass percentage with respect to the total amount, and the particle size distribution was determined. With respect to this particle size distribution, by calculating cumulative mass percentages of the retained on the sieves in descending order of particle size, the relationship between the aperture of the sieve and the cumulative value of the mass percentage of the water-absorbing resin particles retained on the sieve was plotted on a logarithmic probability paper. The plotted points on the probability paper were connected with a straight line, and a particle size corresponding to a cumulative mass percentage of 50 mass% was defined as a median particle size.

### <Physiological Saline Absorption Amount under a Load of 4.14 kPa>

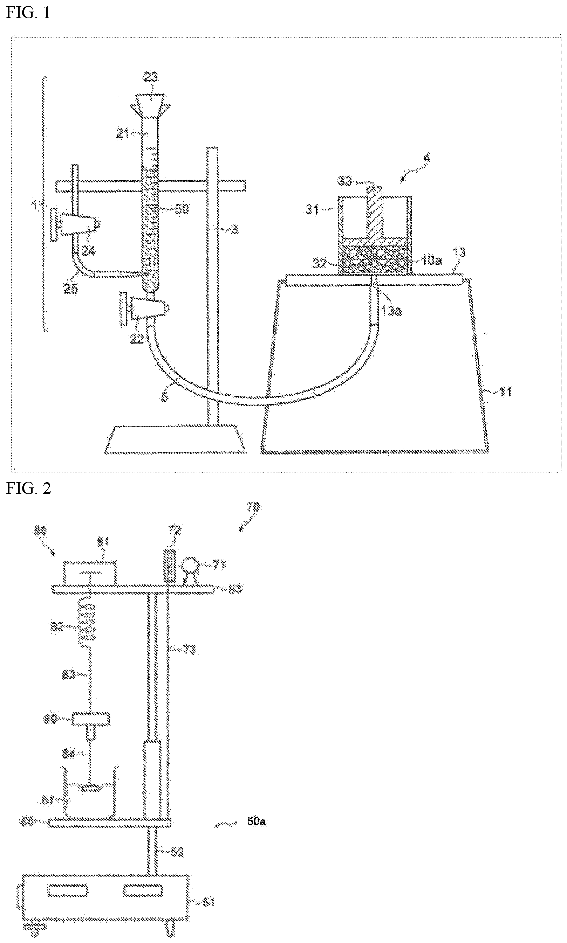

The physiological saline absorption amount under a load of 4.14 kPa (water absorption amount under load) was measured using a measuring apparatus schematically shown in Fig. 1. The measurement was performed twice for one kind of water-absorbing resin particles, and the average value was obtained. The measuring apparatus includes a burette portion 1, a clamp 3, a conduit 5, a rack 11, a measuring table 13, and a measuring unit 4 placed on the measuring table 13. The burette portion 1 includes a burette tube 21 with printed scale, a rubber stopper 23 for hermetically sealing an upper opening of the burette tube 21, a cock 22 connected to a tip of a lower portion of the burette tube 21, and an air introduction tube 25 connected to a lower portion of the burette tube 21, and a cock 24. The burette portion 1 is fixed by the clamp 3. The flat plate-shaped measuring table 13 has a through hole 13a having a diameter of 2 mm formed in a central portion thereof, and is supported by the rack 11 that is height adjustable. The through hole 13a of the measuring table 13 and the cock 22 of the burette portion 1 are connected by the conduit 5. The inner diameter of the conduit 5 is 6 mm.

The measuring unit 4 includes a cylinder 31 made of PLEXIGLAS, a polyamide mesh 32 bonded to one opening of the cylinder 31, and a weight 33 movable in the vertical direction in the cylinder 31. The cylinder 31 is placed on the measuring table 13 with the polyamide mesh 32 interposed therebetween. The inner diameter of the cylinder 31 is 20 mm. The aperture of the polyamide mesh 32 is 75 µm (200 mesh). The weight 33 has a diameter of 19 mm and a mass of 119.6 g, and a load of 4.14 kPa (0.6 psi) can be applied to the water-absorbing resin particles 10a uniformly disposed on the polyamide mesh 32, as described later.

First, the cock 22 and the cock 24 of the burette portion 1 were closed, and 0.9 mass% physiological saline adjusted to 25°C was put into the burette tube 21 through the opening at the upper portion of the burette tube 21. Next, the upper opening of the burette tube 21 was sealed with the rubber stopper 23, and then the cock 22 and the cock 24 were opened. The inside of the conduit 5 was filled with 0.9 mass% saline 50 such that air bubbles did not enter. The height of the measuring table 13 was adjusted such that the height of the water surface of the 0.9 mass% saline reaching the inside of the through hole 13a was the same as the height of the upper surface of the measuring table 13. After the adjustment, the height of the water surface of the 0.9 mass% saline 50 in the burette tube 21 was determined by reading the scale of the burette tube 21, and the position was defined as a 0 point (a read at 0 second).

In the measuring unit 4, 0.10 g of water-absorbing resin particles 10a were uniformly disposed on the polyamide mesh 32 in the cylinder 31, the weight 33 was disposed on the water-absorbing resin particles 10a, and the cylinder 31 was installed such that the central portion thereof coincided with the opening of the conduit at the central portion of the measuring table 13. The decrease amount of the physiological saline in the burette tube 21 (that is, the amount of physiological saline absorbed by water-absorbing resin particles 10a) Wc (ml) was read at 60 minutes after the water-absorbing resin particles 10a started to absorb the physiological saline from the conduit 5, and the physiological saline absorption capacity under a load of 4.14 kPa of the water-absorbing resin particles 10a was calculated by the following equation. 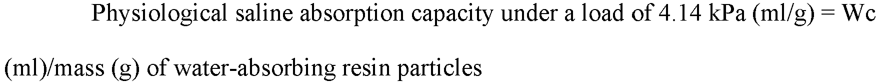 Physiological saline absorption capacity under a load of 4.14 kPa (ml/g) = Wc (ml)/mass (g) of water-absorbing resin particles

### <BET Specific Surface Area>

Water-absorbing resin particles to be measured were adjusted to have a particle size passed through a sieve with an aperture of 400 µm and retained on a sieve with an aperture of 300 µm, and used for measurement of the specific surface area. Next, 10 g of the classified sample was dispersed in 100 g of ethanol, washed with an ultrasonic washer (US-103, manufactured by SND Co., Ltd) for 5 minutes, and then filtered through a sieve with an aperture of 300 µm. The same washing operation was performed two more times to obtain a measurement sample washed three times in total. This sample was dried at 100°C for 16 hours under degassing conditions: heating and vacuum evacuation. Thereafter, an adsorption isotherm was measured at a temperature of 77 K by a method using krypton gas as an adsorption gas with a specific surface area analyzer (AUTOSORB-1, manufactured by Quantachrome Instruments), and a specific surface area was determined from a multipoint BET plot, and taken as the BET specific surface area of the water-absorbing resin particles.

### [Preparation of Activated Carbon]

### (Activated Carbon A)

Activated carbon (Osaka Gas Chemicals Co., Ltd., product name: FP-3) having a BET specific surface area of 1619 m²/g, a median particle size of 38 µm, a residue on ignition of 0.1%, a loss on drying of 0.8%, an iodine adsorption amount of 1560 mg/g, a pH of 7.1, and a crushed shape was prepared. This was designated as activated carbon A.

### (Activated Carbon B)

Activated carbon (Osaka Gas Chemicals Co., Ltd., product name: FPG-1) having a BET specific surface area of 1495 m²/g, a median particle size of 7 µm, a residue on ignition of 3.2%, a loss on drying of 3.4%, an iodine adsorption amount of 1600 mg/g, a pH of 10.2, and a crushed shape was prepared. This was designated as activated carbon B.

### (Activated Carbon C)

Activated carbon (Osaka Gas Chemicals Co., Ltd., product name: LH2c 32/60SS) having a BET specific surface area of 1445 m²/g, a median particle size of 456 µm, a residue on ignition of 0.3%, a loss on drying of 1.0%, an iodine adsorption amount of 1510 mg/g, a pH of 8.3, and a crushed shape was prepared. This was designated as activated carbon C.

### (Activated Carbon D)

The activated carbon C was placed in an electric mixer (IJM-M800-W, manufactured by IRIS OHYAMA Inc.) and pulverized for 5 minutes. Next, the JIS standard sieves were combined in order of a sieve with an aperture of 106 µm, a sieve with an aperture of 75 µm, and a receptacle, and the pulverized activated carbon was put into the combined uppermost sieve and shaken for 10 minutes using a rotating and tapping shaker to perform classification. Thereafter, the activated carbon passed through a sieve with an aperture of 106 µm and retained on the sieve with an aperture of 75 µm was collected. The collected activated carbon had a median particle size of 90 µm and a BET specific surface area of 1608 m²/g. This was designated as activated carbon D.

### [Evaluation of Activated Carbon]

### <Median Particle Size of Activated Carbon (Laser Diffraction)>

The median particle size (D50 (median diameter), volume-based) of the activated carbon used was measured with a laser diffraction particle size distribution analyzer (SALD 2300, manufactured by Shimadzu Corporation).

### <Measurement of BET Specific Surface Area>

Using a pretreatment device (BELPREP VAC II, manufactured by MicrotracBEL Corp.), 0.1 g of the activated carbon to be measured was dried at 60°C for 24 hours under degassing conditions: heating and vacuum evacuation. Thereafter, an adsorption isotherm was measured at a temperature of 77 K by a method using nitrogen gas as an adsorption gas with a specific surface area analyzer (BELSORP MINI II, manufactured by MicrotracBEL Corp.), and a specific surface area was determined from a multipoint BET plot, and taken as the BET specific surface area of the activated carbon.

### [Production of Water-Absorbing Resin Composition]

### <Example 1>

The activated carbon A, 0.1 parts by mass, was added to 100 parts by mass of the water-absorbing resin particles obtained in Production Example 1, and the mixture was mixed for 30 minutes (conditions: revolution speed 50 rpm, rotational speed 50 rpm) using a cross rotary mixer manufactured by Meiwa Kogyo Co., Ltd., to obtain a water-absorbing resin composition.

### <Example 2>

The activated carbon A, 0.3 parts by mass, was added to 100 parts by mass of the water-absorbing resin particles obtained in Production Example 1, and the mixture was mixed for 30 minutes (conditions: revolution speed 50 rpm, rotational speed 50 rpm) using a cross rotary mixer manufactured by Meiwa Kogyo Co., Ltd., to obtain a water-absorbing resin composition.

### <Example 3>

The activated carbon A, 0.5 parts by mass, was added to 100 parts by mass of the water-absorbing resin particles obtained in Production Example 1, and the mixture was mixed for 30 minutes (conditions: revolution speed 50 rpm, rotational speed 50 rpm) using a cross rotary mixer manufactured by Meiwa Kogyo Co., Ltd., to obtain a water-absorbing resin composition.

### <Example 4>

The activated carbon B, 0.1 parts by mass, was added to 100 parts by mass of the water-absorbing resin particles obtained in Production Example 1, and the mixture was mixed for 30 minutes (conditions: revolution speed 50 rpm, rotational speed 50 rpm) using a cross rotary mixer manufactured by Meiwa Kogyo Co., Ltd., to obtain a water-absorbing resin composition.

### <Reference Example 5>

The activated carbon C, 0.1 parts by mass, was added to 100 parts by mass of the water-absorbing resin particles obtained in Production Example 1, and the mixture was mixed for 30 minutes (conditions: revolution speed 50 rpm, rotational speed 50 rpm) using a cross rotary mixer manufactured by Meiwa Kogyo Co., Ltd., to obtain a water-absorbing resin composition.

### <Reference Example 6>

The activated carbon D, 0.1 parts by mass, was added to 100 parts by mass of the water-absorbing resin particles obtained in Production Example 1, and the mixture was mixed for 30 minutes (conditions: revolution speed 50 rpm, rotational speed 50 rpm) using a cross rotary mixer manufactured by Meiwa Kogyo Co., Ltd., to obtain a water-absorbing resin composition.

### <Comparative Example 1>

The water-absorbing resin particles obtained in Production Example 1 were used as Comparative Example 1.

### [Evaluation of Water-Absorbing Resin Composition]

### <Test of Gel That Absorbed Artificial Urine>

A gel (swollen gel) of the water-absorbing resin composition that had absorbed water using artificial urine was tested. As will be described in detail below, the gel strength and the determination result after standing at 40°C for 14 hours were used as indexes for evaluation of stability during normal use, and the gel strength and the determination result after standing at 70°C for 24 hours were used as indexes for evaluation of gel separability during waste treatment.

### (Preparation of Artificial Urine)

Artificial urine having the following composition was prepared.
Urea: 20.0 g
Sodium chloride: 8.0 g
Calcium chloride dihydrate: 0.3 g
Magnesium sulfate heptahydrate: 0.8 g
L (+)-Ascorbic acid: 0.3 g
Ion exchange water: 970.9 g

### (Preparation of Swollen Gel)

The artificial urine, 49.0 g, was weighed out in a beaker having an internal volume of 100 mL, and a magnetic stirrer bar (8 mmφ × 30 mm) was put in the beaker. The beaker was placed on a magnetic stirrer (HS-30D, manufactured by iuchi Corporation), and the magnetic stirrer bar was rotated at 600 rpm. Next, 1.00 g of the water-absorbing resin composition was put into a beaker under stirring, and the mixture was stirred until the rotating vortex disappeared and the liquid level became horizontal, thereby preparing a swollen gel as a measurement sample. Immediately after preparing the swollen gel, the beaker containing the swollen gel was covered with a wrap (DIAWRAP, manufactured by Mitsubishi Chemical Corporation).

### (Measurement of Gel Strength)

The gel strength at each temperature and after the standing time was measured using an apparatus based on the measurement principle shown in Fig. 2. The apparatus illustrated in Fig. 2 includes a support portion 50a, a movable base plate 60, a drive unit 70 for driving the movable base plate 60, and a measuring unit 80. In the support portion 50, a rack 53 is fixed to an upper portion of a support column 52 erected on a support base 51. The movable base plate 60 is attached to the support column 52 so as to move up and down. A measurement sample (gel) 61 can be mounted on the movable base plate 60. A pulse motor 71 is mounted on the rack 53, and rotates a pulley 72 to move the movable base plate 60 up and down via a wire 73. In the measuring unit 80, a pressure sensitive shaft 84 is attached to a load cell 81 for measuring distortion caused by deformation with a precision spring 82 and a connection shaft 83 interposed therebetween. The disk-equipped pressure sensitive shaft 84 has a disk at the tip. Depending on the measurement conditions, the diameter of the disk can be varied. A weight 90 can be mounted above the disk-equipped pressure sensitive shaft 84. The operating principle of the apparatus for measuring gel strength is as follows. The precision spring 82 is fixed to the load cell 81 (stress detector) above, the disk-equipped pressure sensitive shaft 84 is connected to the lower side, and a predetermined weight 90 is placed thereon, which is suspended vertically. The movable base plate 60 on which a measurement sample 61 is placed rises at a constant speed by the rotation of the pulse motor 71. A constant speed load is applied to the sample 61 via the spring 82, strain generated by deformation is measured by the load cell 81, and hardness is measured and calculated. The gel strength value (N/m²) was measured using Curdmeter-MAX (product number: ME-500, manufactured by Aska Instruments Co., Ltd.) under conditions of the temperature and the standing time of each of the following (Initial Value of Gel Strength), (Gel Strength after Standing at 40°C for 14 Hours), and (Gel Strength after Standing at 70°C for 24 Hours), with a pressure sensitive shaft disk of 16 mmφ, a load of 400 g, a speed of 7 seconds/inch, and a viscous mode setting.

### (Initial Value of Gel Strength)

The swollen gel was allowed to stand in an environment at a temperature of 25 ± 2°C and a relative humidity of 50 ± 10% for 60 minutes after preparation, and then the gel strength (initial value of gel strength) was measured by the above-described method.

### (Gel Strength after Standing at 40°C for 14 Hours)

The swollen gel was allowed to stand in a thermo-hygrostat (LHU-113, manufactured by ESPEC CORP.) set at a temperature of 40 ± 2°C and a relative humidity of 60 ± 10% for 14 hours after preparation, and then the gel strength was measured by the above-described method.

### (Gel Strength Change 1 and Evaluation of Gel Stability)

From the obtained gel strength initial value and gel strength 14-hour value, the degree of change 1 (%) was calculated by the following formula. The gel stability was higher as the degree of change 1 was lower, and the stability of the gel was determined from the numerical value of the degree of change 1 using the following criteria. 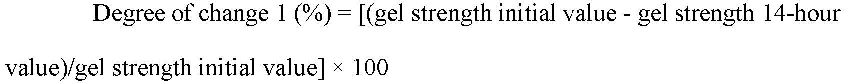 Degree of change 1 (%) = [(gel strength initial value - gel strength 14-hour value)/gel strength initial value] × 100

### <Criteria for Gel Stability>

Degree of change 1 is less than 10%: good
Degree of change 1 is 10% or more: poor

### (Gel Strength after Standing at 70°C for 24 Hours)

The swollen gel was allowed to stand in a thermo-hygrostat (LHU-113, manufactured by ESPEC CORP.) set at a temperature of 70 ± 2°C and a relative humidity of 60 ± 10% for 24 hours after preparation, and then the gel strength was measured by the above-described method.

### (Gel Strength Change 2 and Evaluation of Gel Separability)

From the obtained gel strength initial value and gel strength 24-hour value, the degree of change 2 (%) was calculated by the following formula. The gel became softer as the degree of change 2 was higher, and the ease of gel separating operation was determined from the numerical value of the degree of change 2 using the following criteria. 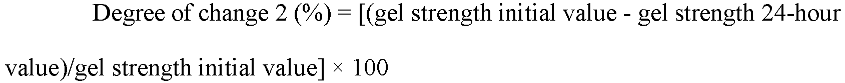 Degree of change 2 (%) = [(gel strength initial value - gel strength 24-hour value)/gel strength initial value] × 100

### <Criteria for Gel Separability>

Degree of change 2 is less than 15%: poor
Degree of change 2 is 15% or more: good

**[Table 1]**

| | Water-absorbing resin particles | | | | Activated carbon | | | Evaluation of water-absorbing resin composition | | | | | | |
|---|---|---|---|---|---|---|---|---|---|---|---|---|---|---|
| | | | | | | | | Initial value (25°C, 1 h) | During normal use (40°C, 14 h) | | | Gel separating operation (70°C, 24 h) | | |
| | Physiological saline absorption amount (g/g) | Median particle size (µm) | Physiological saline absorption amount under a load of 4.14 kPa (mL/g) | BET specific surface area (m²/g) | Median particle size (µm) | BET specific surface area (m²/g) | Content with respect to 100 parts by mass of water-absorbing resin particles (parts by mass) | Gel strength (N/m²) | Gel strength (N/m²) | Degree of gel strength change 1 (%) | Gel stability | Gel strength (N/m²) | Degree of gel strength change 2 (%) | Gel separability |
| Example 1 | 63 | 346 | 21 | 0.033 | 38 | 1619 | 0.1 | 4162 | 4126 | 1 | Good | 3521 | 15 | Good |
| Example 2 | 63 | 346 | 21 | 0.033 | 38 | 1619 | 0.3 | 3942 | 3921 | 1 | Good | 3176 | 19 | Good |
| Example 3 | 63 | 346 | 21 | 0.033 | 38 | 1619 | 0.5 | 3980 | 3982 | 0 | Good | 2421 | 39 | Good |
| Example 4 | 63 | 346 | 21 | 0.033 | 7 | 1495 | 0.1 | 3984 | 3963 | 1 | Good | 3029 | 24 | Good |
| Reference Example 5 | 63 | 346 | 21 | 0.033 | 456 | 1445 | 0.1 | 3762 | 3740 | 1 | Good | 3117 | 17 | Good |
| Reference Example 6 | 63 | 346 | 21 | 0.033 | 90 | 1608 | 0.1 | 3775 | 3720 | 1 | Good | 3107 | 18 | Good |
| Comparative Example 1 | 63 | 346 | 21 | 0.033 | - | - | 0 | 3757 | 3729 | 1 | Good | 3615 | 4 | Poor |

### DESCRIPTION OF REFERENCE SIGNS

1: Burette portion
3: Clamp
4: Measuring unit
5: Conduit
10a: Water-absorbing resin particles
11: Rack
13: Measuring table
13a: Through hole
15: Nylon mesh sheet
21: Burette tube
22: Cock
23: Rubber stopper
24: Cock
25: Air introduction tube
31: Cylinder
32: Polyamide mesh
33: Weight
50: Saline
50a: Support portion
51: Support base
52: Support column
53: Rack
60: Movable base plate
61: Measurement sample (gel)
70: Drive unit
71: Pulse motor
72: Pulley
73: Wire
80: Measuring unit
81: Load cell
82: Precision spring
83: Connection shaft
84: Pressure sensitive shaft
90: Weight

## Claims

1. A water-absorbing resin composition comprising water-absorbing resin particles, and activated carbon disposed on surfaces of the water-absorbing resin particles,
wherein the water-absorbing resin particles are water-absorbing resin particles that have been subjected to a surface-crosslinking treatment,
a physiological saline absorption amount of the water-absorbing resin particle as measured by the method described in the description is 30 to 80 g/g, and
the activated carbon has a median particle size, which is measured by the method described in the description, of 1 µm or more and 70 µm or less.

2. The water-absorbing resin composition according to claim 1, wherein a content of the activated carbon is 0.05 parts by mass or more and 1.0 part by mass or less with respect to 100 parts by mass of the water-absorbing resin particles.

3. The water-absorbing resin composition according to claim 1 or 2, wherein the activated carbon has a median particle size, which is measured by the method described in the description, of 1 µm or more and 45 µm or less.

4. An absorber comprising the water-absorbing resin composition according to any one of claims 1 to 3.

5. An absorbent article comprising the absorber according to claim 4.

6. A method for separation treatment of a water-absorbing resin from the absorbent article according to claim 5, the method comprising the step of heat treatment for 23 hours or more in an environment at a temperature of 70°C or higher and a relative humidity of 40% or higher.

## Patentansprüche

1. Wasserabsorbierende Harzzusammensetzung, die Wasser absorbierende Harzpartikel und auf Oberflächen der wasserabsorbierenden Harzpartikel aufgebrachte Aktivkohle umfasst, wobei
die wasserabsorbierenden Harzpartikel wasserabsorbierende Harzpartikel sind, die einer Oberflächen-Vernetzungsbehandlung unterzogen wurden,
das Absorptionsvermögen für physiologische Kochsalzlösung der wasserabsorbierenden Harzpartikel, gemessen durch das in der Beschreibung beschriebene Verfahren, 30 bis 80 g/g beträgt und
die Aktivkohle eine mediane Partikelgröße, gemessen durch das in der Beschreibung beschriebene Verfahren, von 1 µm oder mehr und 70 µm oder weniger aufweist.

2. Wasserabsorbierende Harzzusammensetzung nach Anspruch 1, wobei der Gehalt der Aktivkohle 0,05 Masseteile oder mehr und 1,0 Masseteile oder weniger, bezogen auf 100 Masseteile der wasserabsorbierenden Harzpartikel beträgt.

3. Wasserabsorbierende Harzzusammensetzung nach Anspruch 1 oder 2, wobei die Aktivkohle eine mediane Partikelgröße, gemessen durch das in der Beschreibung beschriebene Verfahren, von 1 µm oder mehr und 45 µm oder weniger aufweist.

4. Absorber, der eine wasserabsorbierende Harzzusammensetzung nach einem der Ansprüche 1 bis 3 umfasst.

5. Absorbierender Gegenstand, der einen Absorber nach Anspruch 4 umfasst.

6. Verfahren zur Trennungsbehandlung eines wasserabsorbierenden Harzzusammensetzung von einem absorbierenden Gegenstand nach Anspruch 5, wobei das Verfahren den Schritt einer Wärmebehandlung über 23 Stunden oder länger in einer Umgebung mit einer Temperatur von 70 °C oder höher und einer relativen Luftfeuchtigkeit von 40 % oder höher umfasst.

## Revendications

1. Composition de résine absorbant l'eau, comprenant des particules de résine absorbant l'eau, et du charbon actif disposé sur les surfaces des particules de résine absorbant l'eau,
dans laquelle les particules de résine absorbant l'eau sont des particules de résine absorbant l'eau qui ont été soumises à un traitement de réticulation de surface,
une quantité d'absorption de solution saline physiologique de la particule de résine absorbant l'eau telle que mesurée par le procédé décrit dans la description est de 30 à 80 g/g, et
le charbon actif présente une taille de particule médiane, qui est mesurée par le procédé décrit dans la description, de 1 µm ou plus et de 70 µm ou moins.

2. Composition de résine absorbant l'eau selon la revendication 1, dans laquelle une teneur du charbon actif est de 0,05 partie en masse ou plus et de 1,0 partie en masse ou moins par rapport à 100 parties en masse des particules de résine absorbant l'eau.

3. Composition de résine absorbant l'eau selon la revendication 1 ou 2, dans laquelle le charbon actif présente une taille de particule médiane, qui est mesurée par le procédé décrit dans la description, de 1 µm ou plus et de 45 µm ou moins.

4. Absorbeur comprenant la composition de résine absorbant l'eau selon l'une quelconque des revendications 1 à 3.

5. Article absorbant comprenant l'absorbeur selon la revendication 4.

6. Procédé de traitement de séparation d'une résine absorbant l'eau à partir de l'article absorbant selon la revendication 5, le procédé comprenant l'étape de traitement thermique pendant 23 heures ou plus dans un environnement à une température de 70°C ou plus et une humidité relative de 40 % ou plus.
